(19) **Europäisches Patentamt / European Patent Office / Office européen des brevets**

(11) **EP 4 109 006 B1**

(12) # EUROPEAN PATENT SPECIFICATION

(45) Date of publication and mention
of the grant of the patent:
**28.08.2024 Bulletin 2024/35**

(51) International Patent Classification (IPC):
**F24F 11/52** (2018.01)  **F24F 13/20** (2006.01)
**G01N 33/00** (2006.01)  **G01N 1/22** (2006.01)

(21) Application number: **22180842.1**

(22) Date of filing: **23.06.2022**

(52) Cooperative Patent Classification (CPC):
**F24F 11/52; F24F 13/20; G01N 1/2273;**
**G01N 33/0022;** F24F 11/89; F24F 2013/207;
F24F 2110/10; F24F 2110/20; F24F 2110/64;
F24F 2110/66; F24F 2110/68; F24F 2110/70;
G01N 15/06; G01N 2001/245; G01N 2015/0046;

(Cont.)

(54) **INDOOR AIR QUALITY MONITOR**

INNENRAUMLUFTQUALITÄTSÜBERWACHUNG

DISPOSITIF DE SURVEILLANCE DE LA QUALITÉ DE L'AIR AMBIANT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **25.06.2021 US 202163214969 P**

(43) Date of publication of application:
**28.12.2022 Bulletin 2022/52**

(73) Proprietor: **Carrier Corporation**
**Palm Beach Gardens, FL 33418 (US)**

(72) Inventor: **BIRNKRANT, Michael J.**
**Whethersfield, CT 06109 (US)**

(74) Representative: **Dehns**
**St. Bride's House**
**10 Salisbury Square**
**London EC4Y 8JD (GB)**

(56) References cited:
**WO-A1-2017/147778  WO-A1-2020/041224**
**WO-A1-2021/011822  GB-A- 2 539 449**

(52) Cooperative Patent Classification (CPC): (Cont.)
Y02B 30/70

**Description**

The present invention relates to an indoor air quality monitor and to a method for making recommendations regarding air quality.

Indoor air quality is often difficult to understand. This may be due, at least in part, to the numerous parameters that can be measured and reported. Even more difficult is understanding what actions can be taken to improve the indoor air quality. Accordingly, there remains a need for an invention that simplifies the understanding and improvement of indoor air quality.

WO 2021/011822 A1 discloses an environment quality monitoring device and an alert configured to indicate an environment quality score of the ambient environment.

GB 2539449 A discloses an air quality monitor comprising sensors to deduce a measure of indoor air quality from the measurements from the sensors.

According to a first aspect an indoor air quality monitor in accordance with claim 1 is provided.

According to another aspect, a method for making recommendations regarding air quality in accordance with claim 11 is provided.

The subject matter, which is regarded as the invention, is particularly pointed out and distinctly claimed in the claims at the conclusion of the specification. The following descriptions of the drawings should not be considered limiting in any way. Certain exemplary embodiments will now be described in greater detail by way of example only and with reference to the accompanying drawings in which:

FIG. 1A is a perspective side view of an exemplary indoor air quality monitor;

FIG. 1B is a cross-sectional side view of the indoor air quality monitor shown in FIG. 1A, the indoor air quality monitor capable of generating an indoor air quality summary for users in certain instances;

FIG. 2 is a chart that displays an exemplary way of determining normalized scores for the parameters measured by the indoor air quality monitor;

FIG. 3 is an exemplary graph of a bi-linear relation for assigning scores to the parameters measured by the indoor air quality monitor;

FIG. 4 is an exemplary block diagram of the indoor air quality monitor shown in FIGS. 1A and 1B in communication with a user device;

FIG. 5A depicts an exemplary display screen on a user device of an exemplary indoor air quality summary;

FIG. 5B depicts an exemplary recommendation and mitigation action that may be displayed on a user device, the exemplary recommendation and mitigation action being generated as a result of the indoor air quality measured by the indoor air quality monitor; and

FIG. 6 is a flow chart of a method for making recommendations regarding the air quality.

Apparatus and associated methods relate to monitoring air quality. At least one of temperature, humidity, and particulate concentrations is measured by an indoor air quality monitor, which may be disposed in a spot location. Air from that spot location is drawn into the housing of the indoor air quality monitor via a fan assembly. The air drawn into the housing is directed past at least one of a temperature sensor, a humidity sensor, and a particulate detector. The air is then expelled back outside the housing through an outlet in a non-planar upper surface of the housing. A processor generates the indoor air quality summary based on the measured temperature, humidity, and/or particulate concentration.

In some embodiments, the non-planar upper surface of the housing has a plurality of apertures through which the air may be drawn in and expelled back out from the housing (e.g., by operating the fan assembly). Accordingly, it should be understood that in certain instances the apertures on the upper surface of the housing may serve as both an inlet and an outlet for the air being measured by the indoor air quality monitor. However, in certain instance, a barrier may be used to separate which apertures are used to draw air into the housing (which may be referred to as inlet apertures) and which apertures are used to expel air outside the housing (which may be referred to as outlet apertures). As mentioned, a barrier within the housing can be used, in cooperation with a configuration of inlet and outlet apertures, to define the airflow path. Such a barrier can be a tube or a wall, for example. In some embodiments, the airflow path directs the air past the one or more air quality sensors before the air engages electronic components that can alter the air quality metrics (e.g., increasing/decreasing the temperature, humidity, etc. of the air). The one or more air quality sensors may be conductively coupled to an electronic board assembly that is mounted vertically within the housing. In some embodiments, the vertically mounted electronic board assembly can function as a barrier to airflow, thereby contributing to defining the airflow path.

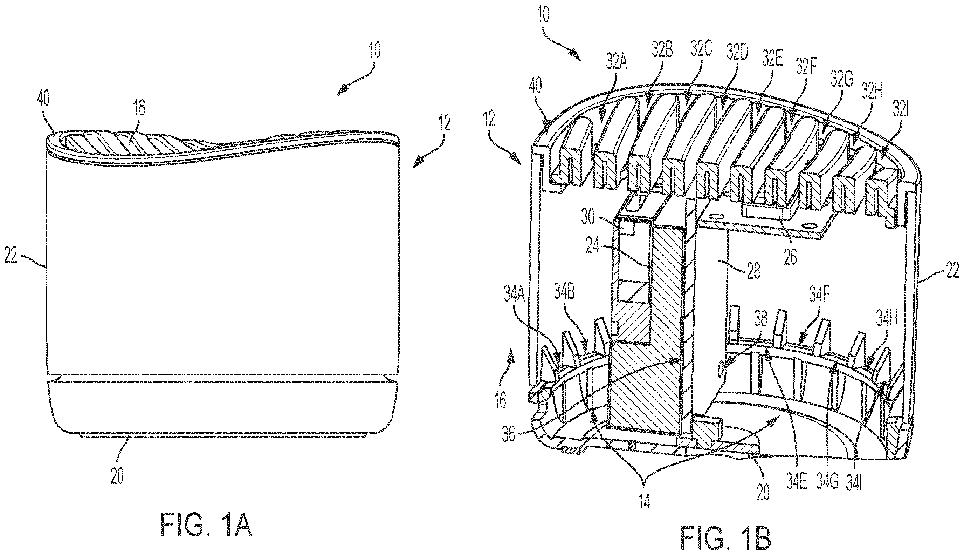

FIGS. 1A and 1B depict a perspective side view and a cross-sectional side view, respectively, of the indoor air quality monitor, which may be disposed at a spot location and capable of generating an indoor air quality summary for a user (e.g., communicate the indoor air quality summary to a user device, such as a smartphone). As will be appreciated, indoor air quality monitor 10 can be situated on surface (e.g., table, floor, etc.) in a room of an environment (e.g., a residential home). Indoor air quality monitor 10 includes housing 12, which defines inside region 14 and outside region 16. Housing 12 has non-planar upper surface 18, lower surface 20 and surrounding sidewalls 22. Lower surface 20 is configured to provide support for air quality monitor 10 upon a surface (e.g., table, floor, etc.) in a room (e.g., kitchen, living room, bedroom, etc.).

Inside of housing 12 are electronic sensor assembly 24 and fan assembly 26. Electronic sensor assembly 24 incudes vertically-mounted electronic board assembly 28 and one or more air quality sensors (e.g., air quality sensor(s) 30). Air quality sensor(s) 30 can be, for example, a temperature sensor, a humidity sensor, a particulate detector, a radon detector, a carbon-dioxide detector, etc. Air quality sensor(s) 30 can be electrically connected to the electronic board assembly 28. In some embodiments, air quality sensor(s) 30 can be directly mounted to electronic board assembly 28. In other embodiments, air quality sensor(s) 30 can be electrically connected to electronic board assembly 28 via conductive wires.

Housing 12 has apertures 32A-32I in non-planar upper surface 18 and at least one aperture in sidewall 22 (which, as shown, may be viewed as one or more circumferential gap, such as apertures 34A-34I, extending around the perimeter of the housing 12). One or more of apertures 32A-32I in non-planar upper surface 18 are referred to as the primary inlet and one or more of apertures 34A-34I in the sidewall 22 are referred to as the secondary inlet. As depicted in FIG. 1B, inside housing 12, in certain instances, barrier 36 separates the inside region 14 into first inside region 14A and second inside region 14B. Air (e.g., from within the environment of which indoor air quality monitor 10 is located) may be drawn from outside region 16 through apertures 32A-32D and 34A-34D into first inside region 14A via fan assembly 26. After the air passes air quality sensor(s) 30, it is expelled into second inside region 14B via barrier aperture 38. The air within second inside region 14B is then expelled back into outside region 16 via apertures 32E-32I and/or 34E-34I. Thus, apertures 32A-32D and 34A-34D function as inlet apertures. Apertures 32E-32I and/or 34E-34I may function as outlet apertures.

It should be appreciated that the non-planar configuration of upper surface 18 (which may be a waveform in certain instances) may help ensure airflow into housing 12. For example, the configuration may be such that the placement of a planar object (such as a book, etc.) on upper surface 18 will not block, at least not completely, apertures 32A-32I (e.g., an air gap may be created between the planar object and the lower portion of the waveform). The non-planar configuration orientation is angled with respect to the apertures. In certain instances, the waveform orientation is angled with respect to the apertures. The wave form orientation angle is non-orthogonal. In some embodiments, like the one depicted in FIGS. 1A and 1B there may be secondary apertures 34A-34I so that should apertures 32A-32I be blocked, airflow through housing 12 could continue.

Air quality sensor(s) 30 is located so as to encounter the air drawn into housing 12 before such air encounters electronic components that might change the metric being measured by air quality sensor(s) 30. For example, air quality sensor(s) 30 is typically mounted near the top of electronic board assembly 28 near where the airflow path is channeled past electronic board assembly 28. If air quality sensor(s) 30 is a temperature sensor, for example, heat-generating electronic components of electronic board assembly 28 may be mounted at locations downstream of where air quality sensor(s) 30 is mounted. Similarly, if air quality sensor(s) 30 is a humidity sensor, it too may be mounted upstream of heat generating electronic components which could affect humidity measurements.

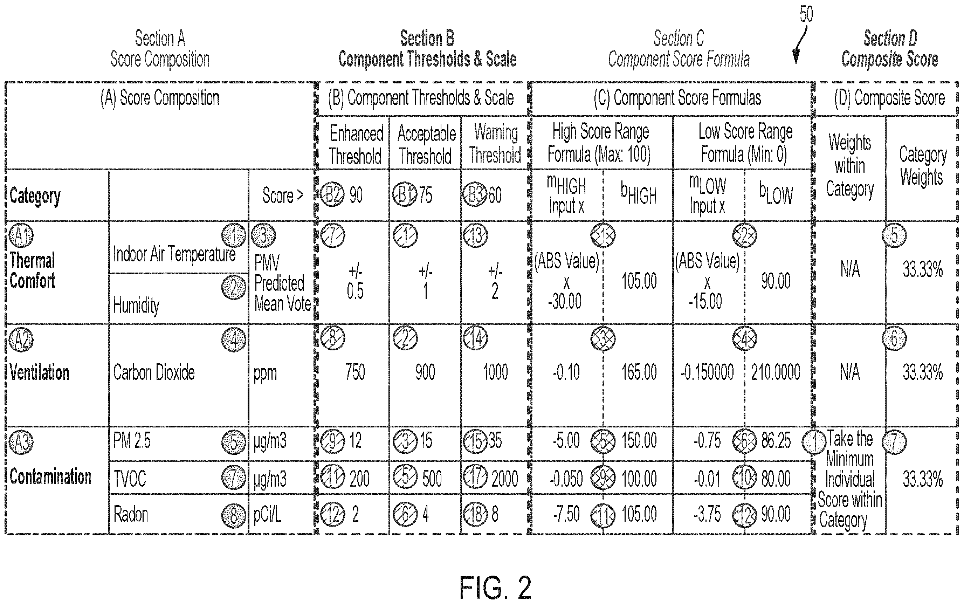

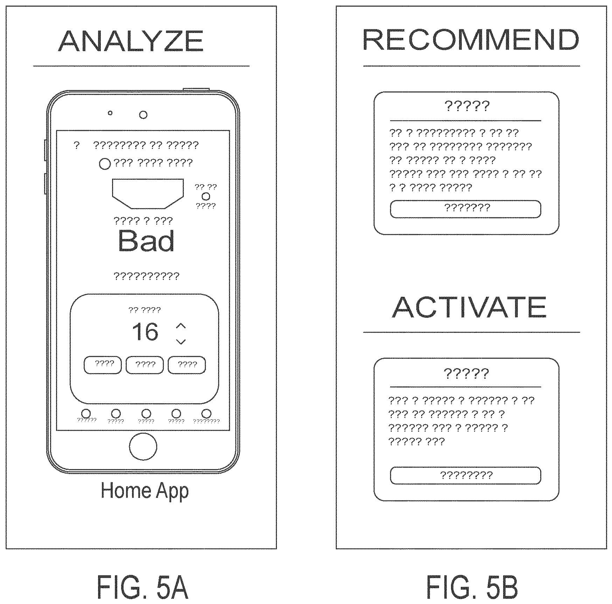

Indoor air quality monitor 10 can perform various operations in response to sensing one or more metrics of air quality. For example, indoor air quality monitor 10 can have light ring 40 configured to display a light signal indicative of the indoor air quality summary (which may be in the form of a different color of light, etc.). Such a summary can be a composite score determined based on the measurement(s) of more than one air quality sensor, as will be described below, with reference to FIG. 2. Indoor air quality monitor 10 can also be configured to transmit the metrics of air quality sensed by the one or more air quality sensors to a remote device, such as, for example, a user's smartphone, as will be described below with reference to FIG. 5. This data can be transmitted to the remote device via Bluetooth, WiFi, or other wireless transmission method. In certain instances, the user's smartphone may be equipped with an indoor air quality monitor application (APP), which can enable a user to visually see the indoor air quality summary from indoor air quality monitor 10. An exemplary display screen of a user device is shown in FIG. 5A.

FIG. 2 is a chart that displays an exemplary way of determining normalized scores for the parameters measured by indoor air quality monitor 10 (e.g., using one or more sensor) Such sensors can provide metrics of air quality that pertain to different categories, such as, for example, thermal comfort, ventilation, and contamination (e.g., via particulates, pollens, poisons, etc.), etc. In FIG. 2, chart 50 includes sections A, B, C, and D. Section A lists the thermal-comfort metrics, the ventilation metrics, and the contamination metrics, in subsections A1, A2, and A3, respectively. In some embodiments, additional categories of atmospheric-environment metrics can be added to those listed in section A.

In sub-section A1, the thermal-comfort metrics are temperature and humidity. Signals indicative of measured temperature and humidity are provided to a room-atmosphere scoring system. In some embodiments the signals indicative of temperature and humidity are used independently, and in other embodiments are used in combination. For example, a Predicted Mean Vote (PMV) can be calculated based on a combination of temperature and humidity measurements. The PMV can be calculated using a proprietary method or any publicly available PMV calculation tool, such as, for example, the pythermalcomfort library, or the Farina, A. PMV Calculator 2015. In some embodiments, additional metrics can be added to the thermal-comfort metrics, such as, for example, air speed. Air speed also can be used either independently or in combination with the temperature metric and/or the humidity metric.

In sub-section A2, the ventilation metrics include only a single metric - carbon dioxide concentration. In other embodiments, additional metrics can be included in the ventilation metrics. For example, another ventilation metric could be a measure or a rate of outside air supplied to the room (e.g., which may be provided in units of cubic feet per minute).

In sub-section A3, the contamination metrics listed are: particulate concentration of particles having a dimension equal to or less than 2.5 microns (PM 2.5); Total Volatile Organic Compound (TVOC) concentration; and Radon concentration. In other embodiments, additional or fewer metrics can be used to determine an overall air quality metric. For example, other toxins or pollutants can be detected by sensors configured to detect such toxins or pollutants. In some embodiments particulate concentration of particles having dimensions equal to or less than 10 microns (PM 10) may be detected and used to determine the overall air quality metric.

Section B of chart 50 discloses how the individual metrics as detected by the various sensors and detectors are normalized. Three different normalization standards are disclosed: i) an enhanced threshold; ii) and acceptable threshold; and iii) a warning threshold. The normalization goes as follows. Each of the individual metrics as detected by the various sensors and detectors are given a score of 75 when the sensed metric is equal to an acceptable threshold. In a similar fashion, each of the individual metrics as detected by the various sensors and detectors are given a score of 90 when the sensed metric is equal to an enhanced threshold. Again, each of the individual metrics as detected by the various sensors and detectors are given a score of 60 when the sensed metric is equal to a warning threshold. Although in the depicted embodiment, the normalized score for metrics equal to the acceptable, enhanced, and warning thresholds is 75, 90, and 60, other normalized scores can be used in other embodiments. The order of the normalized scores, however, will be from highest to lowest from enhanced threshold through acceptable threshold to warning threshold. Furthermore, dissimilar metrics are normalized to the same score, whether it be 75 in the depicted embodiment or some other value, for metrics equal to their respective acceptable threshold.

The acceptable, enhanced, and warning thresholds may be provided by one or more of various standards for a room's atmospheric environment. For example, the International Well Room Institute's V2 Certification Program has published many such thresholds. Other sources can be used to provide guidance pertaining to such thresholds, including government agencies, such as the Environmental Protection Agency (EPA), and international standards, such as the International Standard Organization (ISO). Metrics, for which none of the various standards for a room's atmospheric environment provides such an acceptable level, can leverage experts in the disciplines relevant to provide particular thresholds.

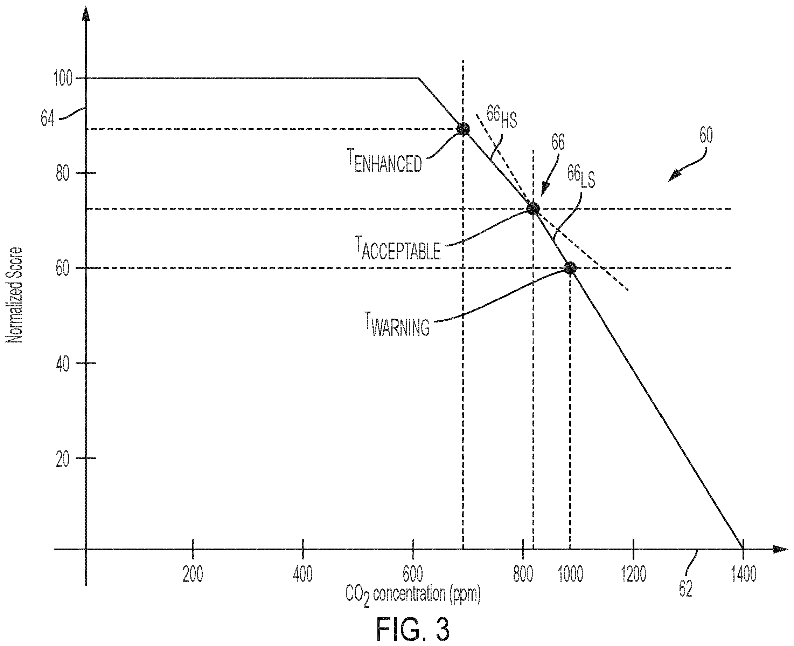

Section B of chart 50 provides exemplary values, obtained from such authorities identified above, for the enhanced, acceptable, and warning thresholds of each atmospheric-environment metric. For example, the acceptable, enhanced, and warning thresholds for carbon dioxide concentration are 900, 750, and 1000 parts per million, respectively, and the acceptable, enhanced, and warning thresholds for TVOC concentration are 500, 200, and 2000 $\mu g/m^3$, respectively. Thus, for any atmospheric-environment metric that is equal to one of the thresholds - the acceptable threshold, the enhanced threshold, or the warning threshold - a score is given - 75, 90, or 60, respectively. For any atmospheric-environment metric that is not equal to one of these thresholds, scores will be computed as will be described below with reference to FIG. 3 and section C of chart 50 of FIG. 2.

FIG. 3 is an exemplary graph of a bi-linear relation for assigning scores to measured parameters measured by indoor air quality monitor 10. Graph 60 is an example of a bilinear relation between one of the parameters included in chart 50 of FIG. 2 - carbon dioxide - and the score assigned thereto. Graph 60 includes horizontal axis 62, vertical axis 64, and concentration-score relation 66. Horizontal axis 62 is indicative of carbon-dioxide concentration in parts per million (ppm). Vertical axis 64 is indicative of normalized score (unitless). Acceptable, enhanced, and warning threshold-score relations are indicated on graph 60, as $T_{ACCEPTABLE}$, $T_{ENHANCED}$ and $T_{WARNING}$, respectively. Concentration-score relation 66 is bilinear with two linear portions - high-score portion $66_{HS}$, and a low-score portion $66_{LS}$. High-score portion $66_{HS}$ can be created by describing a line that passes through both the acceptable threshold-score relation $T_{ACCEPTABLE}$ and the enhanced threshold-score relation $T_{ENHANCED}$. Low-score portion $66_{LS}$ can be created by describing a line that passes through both the acceptable threshold-score relation $T_{ACCEPTABLE}$ and the warning threshold-score relation $T_{WARNING}$.

Concentration-score relation 66 is saturated at score values of 0 and 100 where low-score portion $66_{LS}$ and high-score portion $66_{HS}$ cross such saturation values at $S_{LOW}$ and $S_{HIGH}$ locations on graph 60. Thus, for all carbon-dioxide concentration measurements below 650 ppm, a score of 100 is assigned, and for all carbon-dioxide concentration measurements above 1400 ppm, a score of 0 is assigned. For each of the other atmospheric-environment metrics, a bilinear relation is similarly constructed. Each of high-score portion $66_{HS}$ and low-score portion $66_{LS}$, can be described in standard format:

$$y = mx + b. \tag{1}$$

Here, y represents the score assigned to the atmospheric-environment metric x. the coefficients m and b are given in section C of chart 50. In section C, subscript HIGH identifies the m and b coefficients corresponding to high-score portion $66_{HS}$, and subscript LOW identifies the m and b coefficients corresponding to low-score portion $66_{LS}$.

Section D describes how the individual normalized scores are combined to form a single composite score indicative of the room's atmospheric environment. In the embodiment described by chart 50, a single air quality score is calculated based on the normalized scores of the particulate concentration PM 2.5, the TVOC concentration, and Radon concentration measurements. The minimum of the three normalized measurements of these three concentrations is selected as the normalized score as representative of air quality. In other embodiments the individual normalized metrics can be combined using a weighted average, such as, for example, a weighted arithmetic average. In other embodiments a weighted geometric average can be used to combine individual normalized metrics.

A weighted average of the individual scores representative of thermal comfort, ventilation, and air quality creates a single composite score representative of atmospheric-environment quality for the entire room. Such a composite score can provide a metric, from which a person can readily ascertain quality of the room's atmospheric environment. Furthermore, such a composite score can be compared with a threshold. Should a room's composite score drop below such a threshold, an automated control system can perform remedial actions that are configured to improve the atmospheric environment, and thus improve the composite score. In some embodiments, individual normalized scores can be compared with a threshold for actionable response. For example, if the normalized score for carbon-dioxide concentration were to fall below 75, a remedial action could be triggered. The normalization of scores for different metrics can be done so as to permit a common trigger threshold for remedial action.

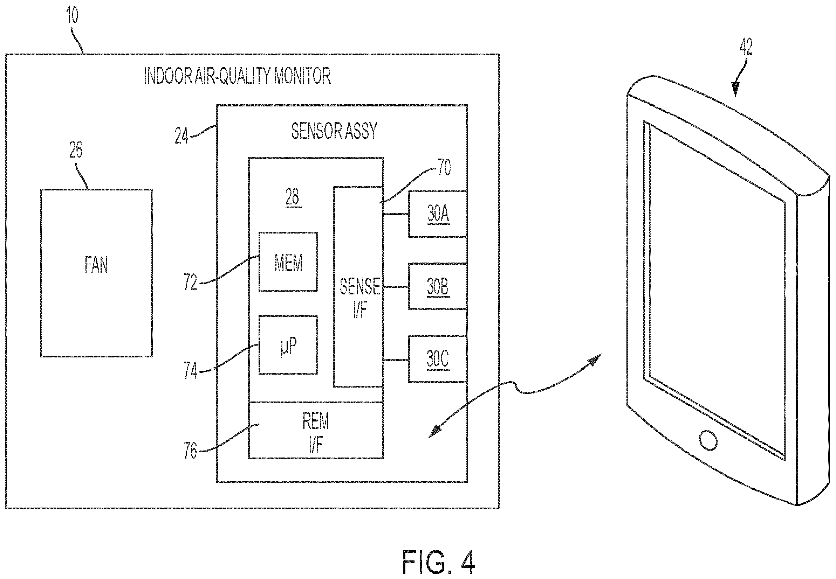

FIG. 4 is a block diagram of indoor air quality monitor 10 in communication with user device 42 (shown here as a smartphone). In FIG. 4, indoor air quality monitor 10 includes electronic sensor assembly 24, fan assembly 26. Electronic sensor assembly 24 include electronic board assembly 28 and air quality sensors 30A-30C. Electronic board assembly 28 includes sensor interface 70, computer readable memory 72; processor 74; remote interface 76; and user interface 78. Processor 74 can be configured to perform operations pertaining to indoor air quality monitor 10. Similarly, computer readable memory 72 can include program instructions $I_{OPERATION}$ pertaining to indoor air quality monitor 10 operations.

To perform functions pertaining to indoor air quality monitor 10 operations, processor 74 may read program instructions $I_{OPERATION}$ from computer readable memory 72, which cause processor 74 to receive signals sensed and/or detected by air quality sensors and/or detectors 30A-30C via sensor interface 76. These received sensor signals can include signals indicative of each of thermal comfort, ventilation, and contamination. Program instructions $I_{OPERATION}$ then may cause processor 74 to convert the received signals indicative of each of thermal comfort, ventilation, and contamination to normalized scores indicative of each of thermal comfort, ventilation, and contamination, respectively. Program instructions $I_{OPERATION}$ then cause processor 74 to combine the normalized scores of each of thermal comfort, ventilation, and contamination to a single composite score indicative of the atmospheric environment in the room. Program instructions $I_{OPERATION}$ then cause processor 74 to send a signal indicative of the composite score to a display device so as to provide notification of the atmospheric environment to a user. Examples of processor 70 can include any one or more of a microprocessor, a controller, a digital signal processor (DSP), an application specific integrated circuit (ASIC), a field-programmable gate array (FPGA), or other equivalent discrete or integrated logic circuitry.

Computer-readable memory 72 can be configured to store information obtained and/or computed during operation of indoor air quality monitor 10. Computer-readable memory 72, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). In some examples, computer-readable memory 72 is a temporary memory, meaning that a primary purpose of computer-readable memory 72 is not long-term storage. Computer-readable memory 72, in some examples, is described as volatile memory, meaning that computer-readable memory 72 do not maintain stored contents when power to atmospheric-environment control system 12 is turned off. Examples of volatile memories can include random-access memories (RAM), dynamic random-access memories (DRAM), static random-access memories (SRAM), and other forms of volatile memories. In some examples, computer-readable memory 72 is used to store program instructions for execution by processor 70. Computer-readable memory 72, in one example, is used by software or applications running on atmospheric-environment control system 12 (e.g., a software program performing room-atmosphere scoring and/or room-atmosphere control) to temporarily store information during program execution.

In some examples, computer-readable memory 72 can also include one or more computer-readable storage media. Computer-readable memory 72 can be configured to store larger amounts of information than volatile memory. Computer-readable memory 72 can further be configured for long-term storage of information. In some examples, computer-readable memory 72 includes non-volatile storage elements. Examples of such non-volatile storage elements can include magnetic hard discs, optical discs, flash memories, or forms of electrically programmable memories (EPROM) or electrically erasable and programmable (EEPROM) memories.

Remote interface 70, in one example, utilizes the communications module to communicate with external devices via one or more networks, such as one or more wireless or wired networks or both. The communications module can be a network interface card, such as an Ethernet card, an optical transceiver, a radio frequency transceiver, or any other type of device that can send and receive information. Other examples of such network interfaces can include Bluetooth, 3G, 4G, and Wi-Fi radio computing devices as well as Universal Serial Bus (USB).

FIG. 5A depicts an exemplary screen on a user device of an exemplary indoor air quality summary. FIG. 5B depicts an exemplary recommendation and mitigation action that may be displayed on a user device, the exemplary recommendation and mitigation action being generated as a result of the indoor air quality measured by indoor air quality monitor 10. In some embodiments, the user can scroll through the various individual air quality metrics sensed by the indoor air quality monitor 10. As shown in FIG. 5A, a display screen graphic on the user device may show a composite score graphic of 16 along with annotation indicating that such a score is one in which the air quality is rated as "Bad". In certain instances, users can navigate between display screens in various manners known in the art, such as for example, touching navigation buttons, etc. to be able to view the details, etc. behind the indoor air quality summary. As shown in FIG. 5B, a display screen of a user device may have text explaining to the user how best to address the air quality score determined. In some embodiments, the user can select to navigate to a website where equipment, which addresses the air quality metric in need of improvement. In some embodiments, the user can also remotely control wirelessly connected air-conditioning equipment via the user interface.

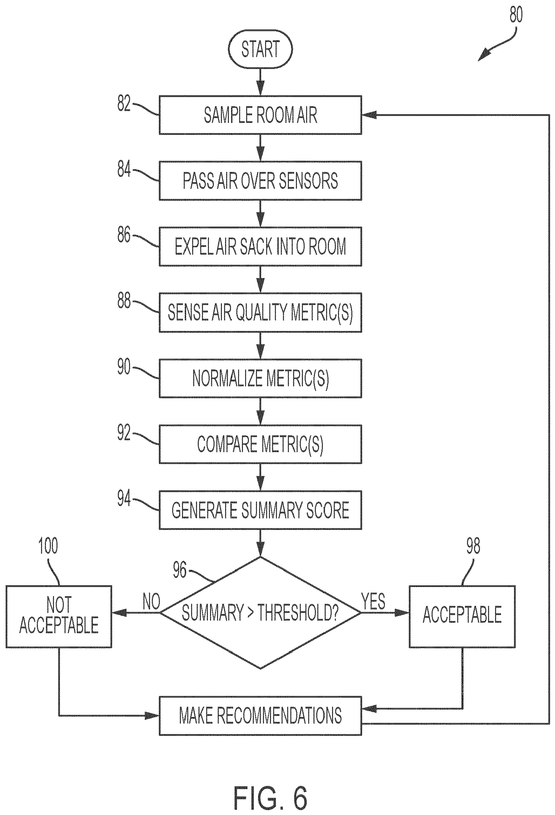

FIG. 6 is a flow chart of a method 80 making recommendations regarding the air quality. It will be appreciated that the method 80 may be performed, at least in part, using the above-described indoor air quality monitor 10. In FIG. 6, method 80 begins at step 82, where air is drawn from outside a housing to inside the housing through an inlet aperture in response to operation of a fan assembly disposed within a housing. At step 84, the air is passed over at least one of a temperature sensor, a humidity sensor, and a particulate detector. At step 86, the air is expelled back through an outlet aperture in a non-planar upper surface of the housing to outside the housing. At step 88, the at least one of the temperature sensor, the humidity sensor, and the particulate detector senses at least one air quality metric of the air drawn into the housing by the fan assembly. At step 90, the processor determines normalized scores for each of the temperature, humidity, and/or particulate concentration sensed at step 88. At step 92, the processor compares the normalized scores with one another so as to determine a worst of the normalized scores determined at step 90.

At step 94, a processor generates an indoor air quality summary or score based on the temperature, humidity, and/or particulate concentration sensed at step 88. In some embodiments, such a summary or score can be a combined score determined as detailed above in the discussion pertaining to FIGS. 2 and 3. In other embodiments, the summary score can be indicative of the worst score determined (i.e., a worst of the normalized scores). At step 96, the summary or score is compared with an air quality threshold. If at step 96, the summary or score is greater than the air quality threshold, method 80 proceeds to step 98, where the processor generates a signal indicative of air quality above the acceptable level. If, however, at step 96, the summary or score is greater than the air quality threshold, method 80 proceeds to step 100, where the processor generates a signal indicative of air quality not above the acceptable level. At both steps 98 and 100, the generated signal (e.g., of air quality above or not above the acceptable level) can be used to provide visual indication or can be sent to a user device.

Regardless of the comparison made at step 96, the method proceeds to step 102, where the processor makes recommendations for improving the air quality, based on the individual sensed air quality metrics, the determined worst metric, and/or the indoor air quality summary or score.

The recommendation, for example could be a presentation of a hyperlink to a website detailing air conditioning equipment that is designed to improve air quality in the manner needed as indicated by the individual sensed air quality metrics, the determined worst metric, and/or the indoor air quality summary or score. After such recommendations, method 80 returns to step 82, and continues to sample the room air.

While the invention has been described with reference to an exemplary embodiment(s), it will be understood by those skilled in the art that various changes may be made without departing from the scope of the appended claims. In addition, many modifications may be made to adapt a particular situation or material to the teachings of the invention without departing from the scope of the invention. Therefore, it is intended that the invention not be limited to the particular embodiment(s) disclosed, but that the invention will include all embodiments falling within the scope of the appended claims.

**Claims**

1. An indoor air quality monitor (10) comprising:

   a housing (12) comprising an upper surface (18) and a lower surface (20), the upper surface (18) comprising a non-planar configuration;
   an electronic board assembly (28) vertically mounted within the housing (12), the electronic board assembly (28) comprising at least one of a temperature sensor, a humidity sensor, and a particulate detector connected thereto; and
   a fan assembly (26) disposed within the housing (12), the fan assembly (26) configured to draw air into the housing (12) over at least one of the temperature sensor, the humidity sensor, and the particulate detector, and expel the air back outside the housing (12),
   **characterised in that**:
   the air is drawn into the housing (12) via at least one of a primary inlet (32A-32I) and a secondary inlet (34E-34I), the primary inlet disposed on the upper surface (18) and the secondary inlet disposed in a sidewall of the housing (12).

2. The indoor air quality monitor (10) of claim 1, wherein the air is expelled back outside the housing (12) via an outlet disposed on the upper surface (18).

3. The indoor air quality monitor (10) of claim 1 or 2, wherein the electronic board assembly (28) comprises a temperature sensor, a humidity sensor, and a particulate detector connected thereto, the temperature sensor configured for generating a signal indicative of the temperature of the air drawn into the housing (12) by the fan assembly (26), the humidity sensor configured for generating a signal indicative of the humidity of the air drawn into the housing (12) by the fan assembly (26), the particulate detector configured for generating a signal indicative of the particulate concentration of the air drawn into the housing (12) by the fan assembly (26).

4. The indoor air quality monitor (10) of claim 3, wherein temperature sensor and the humidity sensor are upstream of the particulate detector.

5. The indoor air quality monitor (10) of claim 3 or 4, further comprising a processor (74) configured to receive the signals and generate an indoor air quality summary, optionally wherein the processor (74) receives a signal indicative of temperature, humidity, and particulate concentration regularly from the temperature sensor, the humidity sensor, and the particulate detector, respectively, and generates the indoor air quality summary each time the temperature, humidity, and particulate concentration are received.

6. The indoor air quality monitor (10) of claim 5, further comprising:
   a light ring (40) disposed about a periphery of the upper surface (18), the light ring (40) configured to display a light signal indicative of the indoor air quality summary.

7. The indoor air quality monitor (10) of claim 5 or 6, further comprising:

   a wireless transmitter configured to wirelessly communicate the indoor air quality summary to a remote receiver, optionally
   wherein the wireless transmitter is configured to wirelessly communicate the temperature, humidity, and particulate concentration to the remote receiver.

8. The indoor air quality monitor (10) of claim 5, 6 or 7, wherein the processor (74) is further configured to compare the temperature, humidity, and particulate concentration with temperature, humidity, and particulate concentration thresholds, respectively, and if at least one of the temperature, humidity, and particulate concentration is less than the respective threshold, then the processor (74) is further configured to determine which of the temperature, humidity and particulate concentration presents the greatest air quality risk.

9. The indoor air quality monitor (10) of any preceding claim, wherein the upper surface (18) comprises a plurality of apertures, wherein the fan assembly (26) is configured to draw air from outside the housing (12) into and also expel air from inside the housing (12) to the outside via the apertures in the upper surface (18).

10. The indoor air quality monitor (10) of any preceding claim, further comprising at least one of:

a Volatile Organic Compound (VOC) sensor electrically connected to the electronic board assembly (28), the VOC sensor configured to generate a signal indicative of the VOC concentration of the air drawn into the housing (12) by the fan assembly (26), wherein temperature sensor and the humidity sensor are upstream of the VOC sensor;
a radon detector electrically connected to the electronic board assembly (28), the radon detector configured to generate
a signal indicative of radon concentration of the air drawn into the housing (12) by the fan assembly (26); and
a carbon-dioxide detector electrically connected to the electronic board assembly (28), the carbon-dioxide detector configured to generate a signal indicative of carbon-dioxide concentration of the air drawn into the housing (12) by the fan assembly (26).

11. A method for making recommendations regarding air quality, the method comprising:

drawing (82), in response to operation of a fan assembly (26) disposed within a housing (12), air from outside the housing (12) to inside the housing (12) through an inlet aperture;
passing (84), in response to operation of the fan assembly (26) disposed within the housing (12), the air over at least one of a temperature sensor, a humidity sensor, and a particulate detector;
expelling (86), in response to operation of the fan assembly (26) disposed within the housing (12), the air back through an outlet aperture in a non-planar upper surface (18) of the housing (12) to outside the housing (12);
sensing (88), via at least one of a temperature sensor, a humidity sensor, and a particulate detector, an air quality metric of the air drawn into the housing (12) by the fan assembly (26); and
generating (94), via a processor, an indoor air quality summary based on the temperature, humidity, and particulate concentration sensed,

characterized by:
drawing the air into the housing (12) via at least one of a primary inlet and a secondary inlet, the primary inlet disposed on the upper surface (18) and the secondary inlet disposed in a sidewall of the housing (12).

12. The method of claim 11, wherein sensing (88) an air quality metric of the air drawn into the housing (12) by the fan assembly (26) comprises sensing, via a temperature sensor, temperature of the air drawn into the housing (12) by the fan assembly (26), the method further comprising:

sensing (88), via a humidity sensor, humidity of the air drawn into the housing (12) by the fan assembly (26); and
sensing (88), via a particulate detector, particulate concentration of the air drawn into the housing (12) by the fan assembly (26).

13. The method of claim 12, further comprising:
determining (90), via the processor, normalized scores for each of the temperature, humidity, and particulate concentration sensed.

14. The method of claim 13, further comprising:
comparing (92), via the processor, the normalized scores with one another so as to determine a worst of the normalized scores.

15. The method of claim 12, 13 or 14, further comprising:
generating (94), via the processor, a signal indicative of the indoor air quality summary.

**Patentansprüche**

1. Innenraumluftqualitätsüberwachung (10), umfassend:

ein Gehäuse (12), das eine obere Fläche (18) und eine untere Fläche (20) umfasst, wobei die obere Fläche (18) eine nichtplanare Konfiguration umfasst;

EP 4 109 006 B1

eine Elektronikplatinenbaugruppe (28), die vertikal innerhalb des Gehäuses (12) montiert ist, wobei die Elektronikplatinenbaugruppe (28) mindestens eines von einem Temperatursensor, einem Feuchtigkeitssensor und einem damit verbundenen Partikeldetektor umfasst; und

eine Lüfterbaugruppe (26), die innerhalb des Gehäuses (12) angeordnet ist, wobei die Lüfterbaugruppe (26) dazu konfiguriert ist, Luft in das Gehäuse (12) über mindestens eines von dem Temperatursensor, dem Feuchtigkeitsensor und dem Partikeldetektor zu saugen und die Luft zurück nach außerhalb des Gehäuses (12) auszustoßen,

**dadurch gekennzeichnet, dass**:

die Luft über mindestens eines von einem primären Einlass (32A-32I) und einem sekundären Einlass (34E-34I) in das Gehäuse (12) gesaugt wird, wobei der primäre Einlass an der oberen Fläche (18) angeordnet ist und der sekundäre Einlass in einer Seitenwand des Gehäuses (12) angeordnet ist.

2. Innenraumluftqualitätsüberwachung (10) nach Anspruch 1, wobei die Luft über einen Auslass, der an der oberen Fläche (18) angeordnet ist, zurück nach außerhalb des Gehäuses (12) ausgestoßen wird.

3. Innenraumluftqualitätsüberwachung (10) nach Anspruch 1 oder 2, wobei die Elektronikplatinenbaugruppe (28) einen Temperatursensor, einen Feuchtigkeitssensor und einen damit verbundenen Partikeldetektor umfasst, wobei der Temperatursensor zum Erzeugen eines Signals konfiguriert ist, das die Temperatur der durch die Lüfterbaugruppe (26) in das Gehäuse (12) gesaugten Luft angibt, wobei der Feuchtigkeitssensor zum Erzeugen eines Signals konfiguriert ist, das die Feuchtigkeit der durch die Lüfterbaugruppe (26) in das Gehäuse (12) gesaugten Luft angibt, wobei der Partikeldetektor zum Erzeugen eines Signals konfiguriert ist, das die Partikelkonzentration der durch die Lüfterbaugruppe (26) in das Gehäuse (12) gesaugten Luft angibt.

4. Raumluftqualitätsmonitor (10) nach Anspruch 3, wobei der Temperatursensor und der Feuchtigkeitssensor stromaufwärts des Partikeldetektors sind.

5. Innenraumluftqualitätsüberwachung (10) nach Anspruch 3 oder 4, ferner umfassend einen Prozessor (74), der dazu konfiguriert ist, die Signale zu empfangen und eine Innenraumluftqualitätszusammenfassung zu erzeugen, wobei optional der Prozessor (74) regelmäßig ein Signal, das Temperatur, Feuchtigkeit und Partikelkonzentration angibt, von dem Temperatursensor, dem Feuchtigkeitssensor bzw. dem Partikeldetektor empfängt und die Innenraumluftqualitätszusammenfassung jedes Mal erzeugt, wenn die Temperatur, Feuchtigkeit und Partikelkonzentration empfangen werden.

6. Innenraumluftqualitätsüberwachung (10) nach Anspruch 5, ferner umfassend:
einen Lichtring (40), der um einen Umfang der oberen Fläche (18) angeordnet ist, wobei der Lichtring (40) dazu konfiguriert ist, ein Lichtsignal anzuzeigen, das die Innenraumluftqualitätszusammenfassung angibt.

7. Innenraumluftqualitätsüberwachung (10) nach Anspruch 5 oder 6, ferner umfassend:

einen drahtlosen Übertrager, der dazu konfiguriert ist, die Innenraumluftqualitätszusammenfassung drahtlos an einen entfernten Empfänger zu kommunizieren,
wobei optional der drahtlose Übertrager dazu konfiguriert ist, die Temperatur, Feuchtigkeit und Partikelkonzentration drahtlos an den entfernten Empfänger zu kommunizieren.

8. Innenraumluftqualitätsüberwachung (10) nach Anspruch 5, 6 oder 7, wobei der Prozessor (74) ferner dazu konfiguriert ist, die Temperatur, Feuchtigkeit und Partikelkonzentration mit Temperatur-, Feuchtigkeits- bzw. Partikelkonzentrationsschwellen zu vergleichen, und falls mindestens eines von der Temperatur, Feuchtigkeit und Partikelkonzentration geringer als die jeweilige Schwelle ist, der Prozessor (74) ferner dazu konfiguriert ist, zu bestimmen, welches von der Temperatur, Feuchtigkeit und Partikelkonzentration das größte Luftqualitätsrisiko darstellt.

9. Innenraumluftqualitätsüberwachung (10) nach einem der vorhergehenden Ansprüche, wobei die obere Fläche (18) eine Vielzahl von Öffnungen umfasst, wobei die Lüfterbaugruppe (26) dazu konfiguriert ist, Luft von außerhalb des Gehäuses (12) hinein zu saugen und zudem Luft von innerhalb des Gehäuses (12) über die Öffnungen in der oberen Fläche (18) nach außerhalb auszustoßen.

10. Innenraumluftqualitätsüberwachung (10) nach einem der vorhergehenden Ansprüche, ferner umfassend mindestens eines von:

einem Sensor für flüchtige organische Verbindungen (Volatile Organic Compound - VOC), der elektrisch mit der Elektronikplatinenbaugruppe (28) verbunden ist, wobei der VOC-Sensor dazu konfiguriert ist, ein Signal zu erzeugen, das die VOC-Konzentration der Luft angibt, die durch die Lüfterbaugruppe (26) in das Gehäuse (12) gesaugt wird, wobei der Temperatursensor und der Feuchtigkeitssensor stromaufwärts des VOC-Sensors sind; einem Radondetektor, der elektrisch mit der Elektronikplatinenbaugruppe (28) verbunden ist, wobei der Radondetektor dazu konfiguriert ist, ein Signal zu erzeugen, das eine Radonkonzentration der Luft angibt, die durch die Lüfterbaugruppe (26) in das Gehäuse (12) gesaugt wird; und

einem Kohlendioxiddetektor, der elektrisch mit der Elektronikplatinenbaugruppe (28) verbunden ist, wobei der Kohlendioxiddetektor dazu konfiguriert ist, ein Signal zu erzeugen, das eine Kohlendioxidkonzentration der Luft angibt, die durch die Lüfterbaugruppe (26) in das Gehäuse (12) gesaugt wird.

11. Verfahren zum Abgeben von Empfehlungen bezüglich einer Luftqualität, wobei das Verfahren Folgendes umfasst:

Saugen (82) von Luft von außerhalb des Gehäuses (12) durch eine Einlassöffnung nach innerhalb des Gehäuses (12) als Reaktion auf einen Betrieb einer Lüfterbaugruppe (26), die innerhalb des Gehäuses (12) angeordnet ist; Leiten (84) der Luft über mindestens eines von einem Temperatursensor, einem Feuchtigkeitssensor und einem Partikeldetektor als Reaktion auf den Betrieb der Lüfterbaugruppe (26), die innerhalb des Gehäuses (12) angeordnet ist;

Ausstoßen (86) der Luft durch eine Auslassöffnung in einer nicht-planaren oberen Fläche (18) des Gehäuses (12) zurück nach außerhalb des Gehäuses (12) als Reaktion auf den Betrieb der Lüfterbaugruppe (26), die innerhalb des Gehäuses (12) angeordnet ist;

Erfassen (88) einer Luftqualitätsmetrik der durch die Lüfterbaugruppe (26) in das Gehäuse (12) gesaugten Luft über mindestens eines von einem Temperatursensor, einem Feuchtigkeitssensor und/oder einem Partikeldetektor; und

Erzeugen (94) einer Raumluftqualitätszusammenfassung basierend auf der erfassten Temperatur, Feuchtigkeit und Partikelkonzentration über einen Prozessor,

**gekennzeichnet durch**:

Saugen der Luft in das Gehäuse (12) über mindestens eines von einem primären Einlass und einem sekundären Einlass, wobei der primäre Einlass an der oberen Fläche (18) angeordnet ist und der sekundäre Einlass in einer Seitenwand des Gehäuses (12) angeordnet ist.

12. Verfahren nach Anspruch 11, wobei das Erfassen (88) einer Luftqualitätsmetrik der durch die Lüfterbaugruppe (26) in das Gehäuse (12) gesaugten Luft Erfassen einer Temperatur der durch die Lüfterbaugruppe (26) in das Gehäuse (12) gesaugten Luft über einen Temperatursensor umfasst, wobei das Verfahren ferner Folgendes umfasst:

Erfassen (88) von Feuchtigkeit der durch die Lüfterbaugruppe (26) in das Gehäuse (12) gesaugten Luft über einen Feuchtigkeitssensor; und

Erfassen (88) einer Partikelkonzentration der durch die Lüfterbaugruppe (26) in das Gehäuse (12) gesaugten Luft über einen Partikeldetektor.

13. Verfahren nach Anspruch 12, ferner umfassend:
Bestimmen (90) normierter Bewertungen für jedes von der erfassten Temperatur, Feuchtigkeit und Partikelkonzentration über den Prozessor.

14. Verfahren nach Anspruch 13, ferner umfassend:
Vergleichen (92) der normierten Bewertungen miteinander, um eine schlechteste der normierten Bewertungen zu bestimmen, über den Prozessor.

15. Verfahren nach Anspruch 12, 13 oder 14, ferner umfassend:
Erzeugen (94) eines Signals, das die Innenraumluftqualitätszusammenfassung angibt, über den Prozessor.

## Revendications

1. Dispositif de surveillance de la qualité de l'air ambiant (10) comprenant :

un boîtier (12) comprenant une surface supérieure (18) et une surface inférieure (20), la surface supérieure (18) comprenant une configuration non plane ;

un ensemble carte électronique (28) monté verticalement à l'intérieur du boîtier (12), l'ensemble carte électronique (28) comprenant au moins l'un d'un capteur de température, d'un capteur d'humidité et d'un détecteur de particules connecté à celui-ci ; et

un ensemble ventilateur (26) disposé à l'intérieur du boîtier (12), l'ensemble ventilateur (26) étant configuré pour aspirer de l'air dans le boîtier (12) sur au moins l'un du capteur de température, du capteur d'humidité et du détecteur de particules, et expulser l'air vers l'extérieur du boîtier (12),

**caractérisé en ce que** :

l'air est aspiré dans le boîtier (12) via au moins l'une d'une entrée primaire (32A-32I) et d'une entrée secondaire (34E-34I), l'entrée primaire étant disposée sur la surface supérieure (18) et l'entrée secondaire étant disposée dans une paroi latérale du boîtier (12).

2. Dispositif de surveillance de la qualité de l'air ambiant (10) selon la revendication 1, dans lequel l'air est expulsé vers l'extérieur du boîtier (12) via une sortie disposée sur la surface supérieure (18).

3. Dispositif de surveillance de la qualité de l'air ambiant (10) selon la revendication 1 ou 2, dans lequel l'ensemble carte électronique (28) comprend un capteur de température, un capteur d'humidité et un détecteur de particules connecté à ceux-ci, le capteur de température étant configuré pour générer un signal indicatif de la température de l'air aspiré dans le boîtier (12) par l'ensemble ventilateur (26), le capteur d'humidité étant configuré pour générer un signal indicatif de l'humidité de l'air aspiré dans le boîtier (12) par l'ensemble ventilateur (26), le détecteur de particules étant configuré pour générer un signal indicatif de la concentration de particules de l'air aspiré dans le boîtier (12) par l'ensemble ventilateur (26).

4. Dispositif de surveillance de la qualité de l'air ambiant (10) selon la revendication 3, dans lequel le capteur de température et le capteur d'humidité sont en amont du détecteur de particules.

5. Dispositif de surveillance de la qualité de l'air ambiant (10) selon la revendication 3 ou 4, comprenant également un processeur (74) configuré pour recevoir les signaux et générer un résumé de la qualité de l'air ambiant, éventuellement dans lequel le processeur (74) reçoit un signal indicatif de température, d'humidité et de concentration de particules régulièrement à partir du capteur de température, du capteur d'humidité et du détecteur de particules, respectivement, et génère le résumé de la qualité de l'air ambiant chaque fois que la température, l'humidité et la concentration de particules sont reçues.

6. Dispositif de surveillance de la qualité de l'air ambiant (10) selon la revendication 5, comprenant également :

un anneau lumineux (40) disposé autour d'une périphérie de la surface supérieure (18), l'anneau lumineux (40) étant configuré pour afficher un signal lumineux indicatif du résumé de la qualité de l'air ambiant.

7. Dispositif de surveillance de la qualité de l'air ambiant (10) selon la revendication 5 ou 6, comprenant également :

un émetteur sans fil configuré pour communiquer sans fil le résumé de la qualité de l'air ambiant à un récepteur distant, éventuellement

dans lequel l'émetteur sans fil est configuré pour communiquer sans fil la température, l'humidité et la concentration de particules au récepteur distant.

8. Dispositif de surveillance de la qualité de l'air ambiant (10) selon la revendication 5, 6 ou 7, dans lequel le processeur (74) est en outre configuré pour comparer la température, l'humidité et la concentration de particules avec des seuils de température, d'humidité et de concentration de particules, respectivement, et si au moins l'une de la température, l'humidité et la concentration de particules est inférieure au seuil respectif, alors le processeur (74) est en outre configuré pour déterminer laquelle de la température, de l'humidité et de la concentration de particules présente le plus grand risque pour la qualité de l'air.

9. Dispositif de surveillance de la qualité de l'air ambiant (10) selon une quelconque revendication précédente, dans lequel la surface supérieure (18) comprend une pluralité d'ouvertures, dans lequel l'ensemble ventilateur (26) est configuré pour aspirer l'air de l'extérieur du boîtier (12) dans et également expulser l'air de l'intérieur du boîtier (12) vers l'extérieur via les ouvertures dans la surface supérieure (18).

10. Dispositif de surveillance de la qualité de l'air ambiant (10) selon une quelconque revendication précédente, comprenant également au moins l'un des éléments suivants :

un capteur de composés organiques volatils (COV) connecté électriquement à l'ensemble carte électronique (28), le capteur de COV étant configuré pour générer un signal indicatif de la concentration en COV de l'air aspiré dans le boîtier (12) par l'ensemble ventilateur (26), dans lequel le capteur de température et le capteur d'humidité sont situés en amont du capteur de COV ;

un détecteur de radon connecté électriquement à l'ensemble carte électronique (28), le détecteur de radon étant configuré pour générer un signal indicatif de la concentration en radon de l'air aspiré dans le boîtier (12) par l'ensemble ventilateur (26) ; et

un détecteur de dioxyde de carbone connecté électriquement à l'ensemble carte électronique (28), le détecteur de dioxyde de carbone étant configuré pour générer un signal indicatif de la concentration en dioxyde de carbone de l'air aspiré dans le boîtier (12) par l'ensemble ventilateur (26).

11. Procédé de formulation de recommandations concernant la qualité de l'air, le procédé comprenant :

l'aspiration (82), en réponse au fonctionnement d'un ensemble ventilateur (26) disposé à l'intérieur d'un boîtier (12), de l'air depuis l'extérieur du boîtier (12) vers l'intérieur du boîtier (12) à travers une ouverture d'entrée ;

le passage (84), en réponse au fonctionnement de l'ensemble ventilateur (26) disposé à l'intérieur du boîtier (12), de l'air sur au moins l'un d'un capteur de température, d'un capteur d'humidité et d'un détecteur de particules ;

l'expulsion (86), en réponse au fonctionnement de l'ensemble ventilateur (26) disposé à l'intérieur du boîtier (12), de l'air de retour à travers une ouverture de sortie dans une surface supérieure (18) non plane du boîtier (12) vers l'extérieur du boîtier (12) ;

la détection (88), via au moins l'un d'un capteur de température, d'un capteur d'humidité et d'un détecteur de particules, d'une mesure de la qualité de l'air de l'air aspiré dans le boîtier (12) par l'ensemble ventilateur (26) ; et

la génération (94), via un processeur, d'un résumé de la qualité de l'air ambiant basé sur la température, l'humidité et la concentration de particules détectées,

**caractérisé par** :

l'aspiration de l'air dans le boîtier (12) via au moins l'une d'une entrée primaire et d'une entrée secondaire, l'entrée primaire étant disposée sur la surface supérieure (18) et l'entrée secondaire étant disposée dans une paroi latérale du boîtier (12).

12. Procédé selon la revendication 11, dans lequel la détection (88) d'une mesure de la qualité de l'air de l'air aspiré dans le boîtier (12) par l'ensemble ventilateur (26) comprend la détection, via un capteur de température, de la température de l'air aspiré dans le boîtier (12) par l'ensemble ventilateur (26), le procédé comprenant également :

la détection (88), via un capteur d'humidité, de l'humidité de l'air aspiré dans le boîtier (12) par l'ensemble ventilateur (26) ; et

la détection (88), via un détecteur de particules, de la concentration de particules de l'air aspiré dans le boîtier (12) par l'ensemble ventilateur (26).

13. Procédé selon la revendication 12, comprenant également :
la détermination (90), via le processeur, de scores normalisés pour chacune de la température, l'humidité et la concentration de particules détectées.

14. Procédé selon la revendication 13, comprenant également :
la comparaison (92), via le processeur, des scores normalisés les uns aux autres de manière à déterminer le pire des scores normalisés.

15. Procédé selon la revendication 12, 13 ou 14, comprenant également :
la génération (94), via le processeur, d'un signal indicatif du résumé de la qualité de l'air ambiant.

FIG. 1A

FIG. 1B

EP 4 109 006 B1

50

| | Section A — Score Composition | | | Section B — Component Thresholds & Scale | | | Section C — Component Score Formulas | | | | Section D — Composite Score | |
|---|---|---|---|---|---|---|---|---|---|---|---|---|
| **(A) Score Composition** | | | | **(B) Component Thresholds & Scale** | | | **(C) Component Score Formulas** | | | | **(D) Composite Score** | |
| | | | | Enhanced Threshold | Acceptable Threshold | Warning Threshold | High Score Range Formula (Max: 100) | | Low Score Range Formula (Min: 0) | | Weights within Category | Category Weights |
| **Category** | | | Score > | (B2) 90 | (B1) 75 | (B3) 60 | $m_{HIGH}$ Input x | $b_{HIGH}$ | $m_{LOW}$ Input x | $b_{LOW}$ | | |
| (A1) **Thermal Comfort** | Indoor Air Temperature ① ; Humidity ② | ③ | PMV Predicted Mean Vote | ⑦ +/- 0.5 | ① +/- 1 | ⑬ +/- 2 | ⑲ (ABS Value) x -30.00 | 105.00 | ㉒ (ABS Value) x -15.00 | 90.00 | N/A | ⑤ 33.33% |
| (A2) **Ventilation** | Carbon Dioxide | ④ | ppm | ⑧ 750 | ㉒ 900 | ⑭ 1000 | ㉝ -0.10 | 165.00 | ㉔ -0.150000 | 210.0000 | N/A | ⑥ 33.33% |
| (A3) **Contamination** | PM 2.5 ⑤ | µg/m3 | | ⑨ 12 | ③ 15 | ⑮ 35 | -5.00 (⑤) | 150.00 | -0.75 (⑥) | 86.25 | ① Take the Minimum Individual Score within Category | ⑦ 33.33% |
| | TVOC ⑦ | µg/m3 | | ⑪ 200 | ⑤ 500 | ⑰ 2000 | -0.050 (⑨) | 100.00 | -0.01 (⑩) | 80.00 | | |
| | Radon ⑧ | pCi/L | | ⑫ 2 | ⑥ 4 | ⑱ 8 | -7.50 (⑪) | 105.00 | -3.75 (⑫) | 90.00 | | |

FIG. 2

FIG. 3

FIG. 4

EP 4 109 006 B1

## ANALYZE

? ???????? ?? ?????
○ ??? ???? ????

?? ??
○
????

???? ? ???

# Bad

??????????

?? ????

16 ∧
∨

| ???? | ???? | ???? |

○        ○        ○        ○        ○
??????   ?????    ?????    ?????    ????????

Home App

## FIG. 5A

## RECOMMEND

?????

?? ? ????????? ? ?? ??
??? ?? ???????? ???????
?? ????? ?? ? ????
????? ??? ??? ???? ? ?? ??
? ? ???? ?????

???????

## ACTIVATE

?????

??? ? ????? ? ?????? ? ??
??? ?? ?????? ? ?? ?
?????? ??? ? ????? ?
????? ???

????????

## FIG. 5B

FIG. 6

**EP 4 109 006 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021011822 A1
- GB 2539449 A